# EUROPÄISCHE PATENTANMELDUNG

(11) **EP 0 614 648 A1**
(43) Veröffentlichungstag der Anmeldung: **14.09.1994**
(21) Anmeldenummer: 94103397.9
(22) Anmeldetag: 07.03.1994
(51) Int. Cl.: A61B 17/39, A61B 17/28

(54) **Arbeitsteile für chirurische Greif- und Schneideinstrumente**

(30) Priorität: 08.03.1993 DE 4307234
(71) Anmelder: Tomic, Dobrivoje, Dr., D-81549 München (DE)
(72) Erfinder: Tomic, Dobrivoje, Dr., D-81549 München (DE)

(57) **Zusammenfassung**

Arbeitsteile für a) chirurgische Greifinstrumente Vorrichtung zum senkrechter Öffnung und Schliessung der Maulteile und einer Vorrichtung zur Vergrösserung der Maulflächen als multifunktionelle Arbeitsteile zum schonenden und atraumatischen Greifen und Halten von Organen, Gewebe, Gefäße, zum Unterbinden und Nathlegen; und b) die Arbeitsteile für chirurgische Schneideinstrumente bestehend aus zwei Schneideblättern, wobei ein Schneideblatt auch als Dissektionselektrode benützt werden kann und parallel zu der Schneideblattelektrode noch eine Elektrode für die bipolare Koagulation angebracht wird, zum Schneiden, Präparieren und zur bipolaren Koagulation Anwendung findet und diese Greif- und Schneideinstrumenten zur Benützung in der minimal invasiven und intrakorporalen endoskopischen Chirurgie verwendung finden.

## Beschreibung

Die Erfindung betrifft die Schaffung von Arbeitsteilen für chirurgische Greifinstrumente mit senkrechter Öffnung und Schliessung der Maulteile und einer Vorrichtung zur Vergrösserung/Veränderung der Maulfläche(n) und die Schaffung von Arbeitsteilen für chirurgische Schneideinstrumente, wobei ein Schneideblatt auch als Dissektionselektrode zweckgeschaffen wird und parallel zu der Schneideblattelektrode noch eine Elektrode für die bipolare Koagulation angebracht wird zur Benützung in der minimal invasiven Chirurgie, insbesondere bei intrakorporalen endoskopischen chirurgischen Eingriffen als multifunktionelle Arbeitsteile zum Greifen, Halten, Heben, als Löffel, zum Naht-, Ligatur- oder Clips-Anlegen, zur Koagulation, Präparation, Schneiden und anderem Verwendung finden.

In der minimal invasiven Chirurgie und insbesondere bei der intrakorporalen endoskopischen Chirurgie sind die Arbeitsteile der Instrumente in verschiedenen, zweckentsprechenden Formen und Grössen und für die entsprechende Anwendung als Greif- und/oder Schneideinstrumente, zum Naht-, Ligatur- oder Clips-Anlegen, als Löffel, zur Koagulation, Präparation, Schneiden, Greifen, Heben, Halten und anderem längst vorhanden, wobei gerade der Arbeitsteil dieser Instrumente viele Nachteile und Mängel bei der Anwendung, Benützung und Durchführung chirurgischer Eingriffe aufweist.

Besonders grosse Nachteile zeigen die Arbeitsteile bei den bekannten Greifinstrumenten, da ihre Maulteile nur eine Schnabel-Winkel Bewegung bei ihrer Öffnung und Schliessung aufweisen und dadurch ein Organ, Gefäss, Gewebe oder anderes schwer greifbar oder haltbar wird und es dabei oft zum Ausrutschen, Verletzung oder Traumatisierung von Organen und Gewebe kommt. Eine bipolare Koagulation ist nur mit der vorderen Maulteilspitze durchführbar und nicht mit der gesamten Maulteilfläche. Eine Vorrichtung zur zusätzlichen Flächenvergrösserung der Maulteile ist bei den bisher bekannnten Arbeitsteilen der Greifinstrumente nicht vorhanden.

Grosse Nachteile zeigen auch die Arbeitsteile der Schneideinstrumente auf, insbesondere bei Scheren, welche keine zusätzliche Vorrichtung zur bipolaren Koagulation und/oder ein Schneideblatt auch als Dissektionselektrode aufweisen, obwohl sehr oft Blutungen bei Scherenbenützung auftreten. Alle diese Nachteile beeinträchtigen den Operationsablauf erheblich, insbesondere mit zeitraubendem Instrumentenwechsel. Auch viele chirurgische Eingriffe, insbesondere bei der intrakorporalen Chirurgie können wegen der erwähnten Nachteile der bisher bekannten Instrumentenarbeitsteile nicht ausgeführt werden.

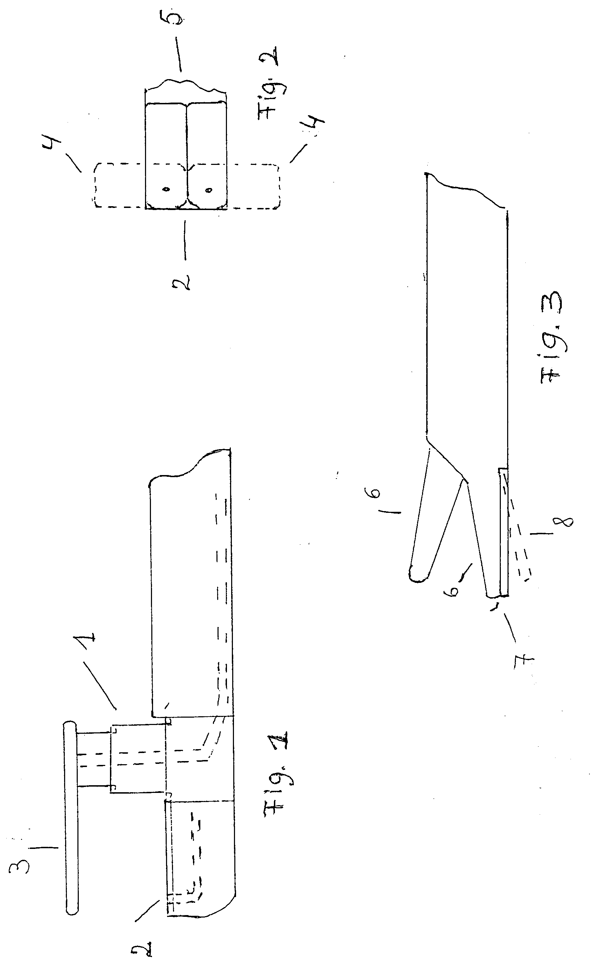

Der Erfindung liegt die Aufgabe zugrunde, a) Arbeitsteile für chirurgische Greifinstrumente mit senkrechter Öffnung und Schliessung der Maulteile (Fig. 1) und einer Vorrichtung zur Flächenerweiterung/Veränderung jedes Maulteiles (Fig. 2) für die Anwendung als multifunktionelle Arbeitsteile in der minimal invasiven Chirurgie, insbesondere bei endoskopischen intrakorporaler Chirurgie anzugeben, bestehend aus zwei parallel zueinander stehenden Maulteilen, wobei jedes Maulteil oder nur das obere Maulteil mit der Vorrichtung zur senkrechten Bewegung gekoppelt ist und jedes Maulteil eine Vorrichtung zu dessen Flächenerweiterung zu eigen haben kann zum Greifen, Halten, Präparieren, Schneiden, zur Koagulation, zum Unterbinden, Naht-, Ligatur oder Clips Anlegen, als Löffel oder anderes, und diese angegebenen Vorrichtungen auch für viele andere Instrumente als Arbeitsteile Anwendung finden; und b) die Arbeitsteile für chirurgische Schneideinstrumente anzugeben (Fig. 3), bestehend aus zwei Schneideblättern, wobei ein Schneideblatt auch die Form und Funktion einer Dissektionselektrode aufweisen kann und eine zusätzliche, parallel zu der Schneideblattelektrode angebracht wird, und als multifunktioneller Arbeitsteil zum Schneiden, Präparieren und zur bipolaren Koagulation Anwendung findet, die eine Vielzahl der bisher benötigten Instrumente mit bekannten Arbeitsteilen sowie Instrumentenwechsel bei der Operation zu vermindern, die operativen Möglichkeiten zu erweitern und den Operationsablauf zu vereinfachen und zu verkürzen, wobei die angegebenen Arbeitsteile beziehungsweise Vorrichtung zur Flächen- und Formausdehnung der Maulteile auch für die Arbeitsteile mit dem Schnabel-Winkel oder anderem Maulbewegungssystem Anwendung findet.

Gegenstand der Erfindung sind die a) Arbeitsteile für chirurgische Greifinstrumente und Scneideinstrumente, wobei der Arbeitsteil bei den Greifinstrumenten aus zwei parallel zueinander stehenden Maulteilen besteht, die Maulteile verschiedene Formen und Grössen aufweisen können und beide Maulteile oder nur der obere Maulteil sich erfindungsgemäss mit einer Vorrichtung senkrecht zur Öffnung und Schliessung der Maulteile bewegt zur Benützung als Greifer, Zange, Löffel, Schere, Krallen, Hacker, Klemme, Halter, mono- der bipolare Elektroden und andere und jeder Maulteil bei allen chirurgischen Greifinstrumenten seine Greiffläche während der Anwendung und jederzeit vergrössern und/oder verändern kan und b) die Arbeitsteile für die chirurgischen Schneideinstrumente, aus zwei Schneideblättern bestehend, ein Schneideblatt zugleich eine Dissektionselektrode aufweist und zudem noch eine zusätzliche parallel zu der Schneideblattelektrode verlaufende Elektrode angebracht werden kann zur Benützung und Anwendung als multifunktioneller Arbeitsteil zum Schneiden, Präparieren und zur bipolaren Koagulation. Alle Instrumente mit erfindungsgemässen Arbeitsteilen können auch eine Saug- und/oder Spritzvorrichtung aufweisen.

Der erfindungsgemässe zweckgeformte Arbeitsteil mit senkrechter Maulteilbewegung für chirurgische Greifinstrumente weist in seiner Grundausführung zwei parallel zueinander stehende Maulteile auf, welche mit dem zweckgeformten Schaft und Handgriff eine Instrumenteneinheit darstellt. Der Schaft ist in seinem Inneren ein Hohlraum durch welchen verschiedene Betätigungsvorrichtungen vom Handgriff bis zum Arbeitsteil verlaufen können. Vorzugsweise kann der untere Maulteil in starrer Verbindung mit dem Schaft sein. Zwischen diesem unteren Maulteil beziehungsweise beider Maulteile und dem distalen Ende des Schaftes befindet sich erfindungsgemäss die Vorrichtung zur senkrechten Bewegung des oberen Maulteiles oder beider Maulteile, welche sich vorzugsweise in einem Schachtähnlichem Hohlraum befinden, in dem sich auch der Halter und Träger beziehungsweise Heber des oberen beweglichen Maulteiles befindet. Der Träger oder Heber kann aus einem Stück/Teil oder aus mehreren Teilen beziehungsweise Segmenten bestehen und mit einem Teleskop- oder Hebesystem das obere Maulteil oder beide Maulteile senkrecht nach oben und unten mittels einer Betätigungsvorrichtung bewegen und so die erfindungsgemässe senkrechte Öffnung und Schliessung der beiden Maulteile bewirken. Einer oder beide Maulteile können verschiedene zweckentsprechende Formen, Grössen sowie Elektroden aufweisen, aus verschiedenen Materialien bestehen, überzogen, isoliert, beschichtet und/oder abnehmbar sein.

Einer oder beide Maulteile können erfindungsgemäss bewegliche Flächen in Form von Flügeln, Blättern, Plättchen, Segmenten oder ähnlichhem zur Ausdehnung/Verformung ihrer Greif- beziehungsweise Maulfläche aufweisen, wobei diese erfindungsgemässe Flächenvergrösserung beziehungsweise -ausdehnung für alle Arbeitsteile chirurgischer Greifinstrumente Anwendung finden kann.

Der erfindungsgemässe Arbeitsteil für chirurgische Schneideinstrumente weist zwei Schneideblätter und eine parallel zu einem Schneideblatt stehende Elektrode auf, welche zusammen mit dem Schaft und Handgriff eine Instrumenteneinheit darstellt. Eines der Schneideblätter ist so geschaffen, dass es auch als Elektrode oder auch als Dissektionselektrode benützt werden kann, wobei seine Spitze entsprechend geformt ist wie ein Haken, Spatel, Kugel oder anderes. Eine zweite Elektrode ist neben der Schneideblattelektrode angebracht und kann in den Schaft versenkt sein oder parallel zur Schneideblattelektrode stehen zur erfindungsgemässen Anwendung bei der bipolaren Koagulation, wobei der so geschaffene erfindungsgemässe Arbeitsteil als multifunktioneller Teil für chirurgische Schneideinstrumente breite Anwendung findet. Schneideblätter und Elektroden können verschiedene Grössen und Formen aufweisen und/oder beschichtet und/oder isoliert sein.

Die Betätigungsvorrichtung für die erfindungsgemässen Arbeitsteile, für die senkrechten Bewegungen des Maulteiles und die Benützung der Elektroden kann auf üblicher bekannter Weise und mit verschiedenen im Gebrauch stehenden Vorrichtungen und Möglichkeiten erfolgen.

Die folgende Zeichnung soll als Beispiel dienen die Erfindung und die Ansprüche näher zu erläutern.

## Patentansprüche

1. Arbeitsteile für chirurgische Greifinstrumente mit senkrechter Bewegung eines oder beider Maulteile und Vorrichtung zum senkrechten Öffnen und Schliessen der Maulteile (1) und einer Vorrichtung zur Vergrösserung und Veränderung der Maulfläche(n) (2) und Maulteile, und Schaffung von Arbeitsteilen für chirurgische Schneideinstrumente, wobei ein Schneideblatt auch als Dissektionselektrode zweckgeschaffen ist und dass parallel zu dieser Schneideblattelektrode noch eine zweite Elektrode für die bipolare Koagulation angebracht wird und alle diese Arbeitsteile in der minimal invasiven Chirurgie, insbesondere bei intrakorporalen endoskopischen Eingriffen ihre Benützung und Anwendung finden, als multifunktionelle Arbeitsteile und Vorrichtungen zum Greifen, Halten, Heben, als Löffel, Kralle, Haken, Zange, kleine Schere, zum Unterbinden, Naht-, Ligatur- oder Clips Anlegen, zur Koagulation, Präparation, Schneiden und anderem, dadurch gekennzeichnet, dass
a) der Arbeitsteil für chirurgische Greifinstrumente (3) oder beide Maulteile mit Vorrichtung zur senkrechten Bewegung beim Öffnen und Schliessen aufweist.
b) der Arbeitsteil für chirurgische Greifinstrumente eine Vorrichtung zur Vergrösserung der Greiffläche aufweisen kann, bevorzugt in Form beweglicher Blätter, Plättchen, Flügel, Segmente oder ähnlichem (4), welche an die Maulteile (5) angebracht werden können.
c) ein Schneideblatt für chirurgische Schneideinstrumente in zweckentsprechender Form und Vorrichtung auch als Elektrode und Dissektionselektrode benützt wird.
d) Der Arbeitsteil für chirurgische Schneideinstrumente aus zwei Schneideblättern (6) besteht wovon ein Schneideblatt gleichzeitig auch eine Elektrode (7) ist und eine zweite Elektrode parallel zu dieser Schneideblattelektrode angebracht ist (8).

2. Arbeitsteile für chirurgische Instrumente nach Anspruch 1, dadurch gekennzeichnet, dass sie verschiedene Grössen und zweckentsprechende Formen aufweisen, aus verschiedenen Materialien, überzogen, isoliert, beschichtet und abnehmbar sein können.

3. Arbeitsteile oder einzelne Vorrichtungen oder Teile nach Anspruch 1 bis 1d, dadurch gekennzeichnet, dass sie für verschiedene chirurgische Instrumente Anwendung finden, sowie eine Saug/Spritzvorrichtung und Elektroden aufweisen können.
